# EUROPEAN PATENT SPECIFICATION

(11) **EP 1 610 785 B1**
(45) Date of publication and mention of the grant of the patent: **22.08.2007**
(21) Application number: 04726223.3
(22) Date of filing: 07.04.2004
(51) Int. Cl.: A61K 31/4453, A61K 31/485, A61P 25/08, A61P 21/02

(54) **PHARMACEUTICAL COMBINATION FOR THE TREATMENT OF SPASTICITY AND/OR PAIN**
PHARMAZEUTISCHE KOMBINATIONSPRÄPARAT ZUR BEHANDLUNG VON SPASTISCHKEIT UND/ODER SCHMERZEN
COMBINAISON PHARMACEUTIQUE POUR LE TRAITEMENT DE L'HYPERTONIE SPASTIQUE ET/OU LA DOULEUR

(30) Priority: 09.04.2003 HU 0300929 P
(43) Date of publication of application: 04.01.2006
(73) Proprietor: RICHTER GEDEON VEGYÉSZETI GYÁR RT, 1103 Budapest X (HU)
(72) Inventor: TIHANYI, Károly, H-1171 Budapest (HU); KOCSIS, Pál, H-1155 Budapest (HU); NÉMETH, György, H-4032 Debrecen (HU); TARNAWA, István, H-1147 Budapest (HU); DALMADI, Balázs, H-1046 Budapest (HU)
(74) Representative: Grof, Palma
(86) International application number: PCT/HU2004/000032
(87) International publication number: WO 2004/089352

(56) References cited:
- WO-A-00/59508
- WO-A-89/05641
- WO-A-89/05642
- WO-A-97/04780
- WO-A-98/07447
- DATABASE WPI Section Ch, Week 197821 Derwent Publications Ltd., London, GB; Class B03, AN 1978-37512A XP002300728 & JP 53 040779 A (NIPPON KAYAKU KK) 13 April 1978 (1978-04-13)
- [Online] Retrieved from the Internet: URL:wwww.ninds.nih.gov/disorders/spasticit y/spasticity.htm>
- [Online] Retrieved from the Internet: URL:www.ninds.nih.gov/disorders/epilepsy/e pilepsy.htm>

## Description

This invention relates to a pharmaceutical combination for the treatment of spasticity and/or pain.

It is known, that tolperisone (chemical name: 2-mehyl-1-(4-methylphenyl)-3-(1-piperidinyl)-1-propanone) is the active ingredient of Mydeton, a product which has been on the market for decades. Tolperisone is a centrally acting muscle relaxant (CMR). It is indicated for the treatment of spasticity and painful reflex muscle spasms associated with various musculoskeletal diseases, for example lumbago, spastic states after spinal or brain trauma, stroke, perinatal head injuries, or sclerosis multiplex, amyotrophic lateral sclerosis, etc.

The mode of action of the centrally acting muscle relaxants can be different. They can act via GABA_{A}, GABA_{B}, alpha₂ or NMDA receptors, while some of them block voltage-dependent Na⁺ and Ca²⁺ channels. Tolperisone is a drug that can be applied safely since it is relatively free from side effects. Its oral bioavailability is low; it is 16-20 % in humans.

Tolperisone and its use as central muscle relaxant is known and disclosed in JP-A-53040779

It should be noted, that there is no unambiguous clinical evidence for the analgesic effect of the tolperisone.

It is well-known, that dextromethorphan (chemical name: (+/-)-3-methoxi-17-methylmorphinan) has a similar structure to codein. Dextromethorphan is mainly used as a cough suppressor in the clinical practice. It is a safe drug, which has few side effects, and it is used in pediatrics, too. There is only one known clinical drug interaction: dextromethorphan can not be administered with MAO-inhibitors.

There are dextromethorphan combination products with ephedrine and acetaminophen on the market, used as bronchodilators and are indicated for the treatment of cold with cough symptoms.

Dextromethorphan is identified as an NMDA type glutamate receptor antagonist drug, but it is known to have other significant effects, too. Its main metabolite is dextrorphan, which is a more effective NMDA antagonist. The therapeutic effects of dextromethorphan are believed to be based on this mechanism.

The above mentioned NMDA antagonism was the theoretical basis underlying the combination of dextromethorphan, with morphine for the treatment of pain symptoms. Dextromethorphan had been expected to block or reverse tolerance to morphine analgesia. However, the clinical trial with this combination proved to be unsuccessful.

We emphasize, that a number of clinical trials had been run in order to prove the intrinsic analgesic effect of dextromethorphan. However, it was found ineffective in the treatment of chronic pain. (Ben-Abraham and Weinbroum, Isr. Med. Assoc. J. 2000. 2, 708.). The molecule was also without effect in relieving acute ischemic pain in humans. (Plesan et al., Acta Anaesthesiol. Scand. 2000. 44, 924.). It was also reported that in an experimental human pain model, dextromethorphan had no effect in the clinically acceptable dose range (Kaippula et al., Pharmacol. Biochem. Behav. 1995. 52, 611.). According to the clinical trials no significant analgesic effect was found with the following combinations: dextromethorphan-NSAID, dextromethorphan-propoxyphene, dextromethorphan-morphine. (Mercandate et al., J. Pain Symptom. Manage. 1998. 16, 317.). In pediatric therapy, the perioperative use of dextromethorphan does not improve morphine or acetaminophen induced analgesia (Rose et al., Anesth. Analg. 1999. 88, 749.).

According to the above mentioned studies dextromethorphan has no proven analgesic effect either by itself or in combination,, and the treatment of pain is not among the indications of dextromethorphan. Its antispastic effect has not been recognized, thus it is not among its indications either.

WO-A-890 5641 discloses combination products of an anticonvulsant and a potentiating amount of dextromethorphan for treating epilepsy.

The ami of our invention is to work out a pharmaceutical combination suitable for the effective treatment of spasticity and pain.

In our first experiments, we investigated the ability of dextromethorphan to increase the efficacy of tolperisone. We emphasize, that the interaction of tolperisone and dextromethorphan has not been studied neither in animal pharmacological experiments nor in human clinical studies. Surprisingly, in spasticity models, our experimental results demonstrated a so far unknown and unexpected potentiating effect when these two marketed pharmaceutical products were co-administered. In addition to this, we recognized a new pharmacological effect of the combination; analgesia, which had not been acknowledged before concerning the individual use of the any of these drugs in the human therapy. On the contrary, as we mentioned above, all human studies with dextromethorphan combinations have failed according to the scientific literature.

According to the aforesaid, our invention is a pharmaceutical combination for the treatment of spasticity and/or pain contain, as active ingredient, compound of formula I, wherein R represents a methyl or ethyl group, in an amount of 70-95 % w/w and dextromethorphan (chemical name: (+/-)-3-methoxi-17-methylmorphinan) in an amount of 5-30 % w/w.

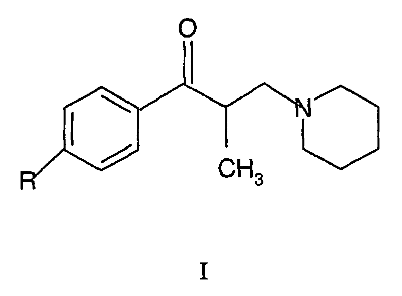

In an advantageous pharmaceutical composition, according to the present invention, tolperisone is present in an amount of 80 to 90% w/w, and dextromethorphan is present in an amount of 10 to 20% w/w.

A unit of the pharmaceutical composition of the present invention preferentially contains 150 mg tolperisone and 30 mg dextromethorphan in admixture with pharmaceutically acceptable carriers.

The composition of this invention is for oral administration.

In order to prove the effectivity of the pharmaceutical combination of the invention the following experiments were carried out:

### A./

The effect of dextromethorphan on muscle relaxant and reflex inhibitory activities of tolperisone

### Method for evaluating muscle relaxant efficacy

### Tremor test in mice

Inhibitory effect on drug-induced tremor in mice is a good indication of muscle relaxant efficacy of compounds in humans. Tremor can be induced by administration of GYKI 20039 (3-(2,6-dichlorophenyl)-2-iminothiazolidine). The method was published by Kocsis P., Tarnawa I., Kovács Gy., Szombathelyi Zs., Farkas S., 2002; Acta Pharmaceut. Hung., 72:49-61; US 5340823,1994, US 5198446, JP 1992270293, EP 0468825, 1990 HU 4647 .

Our experiments revealed that GYKI 20039 induces intensive tremor at 10 mg/kg intraperitoneal dose in mice, which lasts for 30-60 minutes and reaches its maximum between the 4-8th minutes. The mode of action of GYKI 20039 is not entirely clear, but its structural similarity to LON-954, another tremorogen compound, suggests the involvement of the descending dopaminergic and serotoninergic systems (Mohanakumar, K.P., Ganguly, D.K., 1989; Brain Res. Bull. 22: 191-5).

The GYKI 20039 induced tremor can be dose-dependently inhibited by muscle relaxant drugs with different modes of action; therefore it is a suitable method for comparing the muscle relaxant efficacy of drugs. The model has been validated using several different muscle relaxant drugs used in the clinical practice (Table 1). All of them produced dose-dependent inhibitory effect, which correlated well with the clinical antispastic efficacy of the muscle relaxant drugs.

The aim of our experiments was to decide if the effect of tolperisone is influenced by co-administration of dextromethorphan. To clarify this issue, we chose a low dose of dextromethorphan that alone did not inhibit the GYKI 20039 induced tremor.

**Table 1.**

| Muscle relaxant | Tremor inhibitory effect ED₅₀ (mg/kg, i.p.) |
|---|---|
| Tolperisone | 60.0 |
| Eperisone | 51.8 |
| Tizanidine | 0.73 |
| Baclofen | 9.1 |
| Mephenesin | 81.5 |
| Zoxazolamine | 27.4 |
| Diazepam | 0.94 |
| Afloqualon | 11.1 |
| Carisoprodol | 128.8 |
| Memantine | 20.5 |

### Doses of different muscle relaxant drugs causing 50% tremor inhibition (intraperitoneal administration, 15-minute pre-treatment)

Tables 2 and 3 clearly demonstrate that dextromethorphan, tested at different doses, significantly increases the effect of various doses of tolperisone.

Dextromethorphan, administered 15 minutes before GYKI 20039, intraperitoneally, caused a dose-dependent inhibition of the tremor with an ED₅₀ of 24.4 mg/kg. A 10-minute pre-treatment with 10 mg/kg of dextromethorphan caused no significant tremor inhibition, but significantly increased the inhibitory action of 40 or 60 mg/kg tolperisone. (Table. 3.). The ED₅₀ value for the tremor inhibitory activity of tolperisone alone was 55.0 ± 7.2 mg/kg, while when combined with dextromethorphan at a ratio of 5:1 it had an ED₅₀ of 33.0 ± 0.7 mg/kg. At a ratio of 10:1, the ED₅₀ was 26.6 ± 4.7 mg/kg, while at 20:1 the ED₅₀ was 34.4 ± 6.8 mg/kg for tolperisone.

**Table 2.**

| Tolperisone (mg/kg, i.p.) | Dextromethorphan (mg/kg, i.p.) | Inhibition of tremor (%) | S.E.M | p |
|---|---|---|---|---|
| 60 | 0 | 48.3 | 7.4 | |
| 60 | 3 | 76.7 | 7.0 | 0.00165 |
| 60 | 6 | 79.6 | 4.8 | 0.000878 |
| 60 | 12 | 84.2 | 3.4 | 0.000265 |

The tremor inhibitory action of 60 mg/kg tolperisone alone and in the presence of dextromethorphan at different ratios (20:1; 10:1 and 5:1). Dextromethorphan significantly increased the effect of tolperisone. (p=0.00045; ANOVA). The post hoc test (Duncan's multiple range test) compared the result of each group treated with the dextromethorphan-tolperisone combination to that of the group treated with the corresponding dose of tolperisone alone.

**Table 3.**

| Treatment (i.p.) | Inhibition of tremor (control %) | | N |
|---|---|---|---|
| | Mean | S.E.M. | |
| 10 mg/kg dextromethorphan | 8 | 7 | 20 |
| 40 mg/kg tolperisone | 27 | 6 | 20 |
| 40 mg/kg tolperisone + 10 mg/kg dextromethorphan | 59 | 3 | 20 |
| 60 mg/kg tolperisone | 48 | 7 | 10 |
| 60 mg/kg tolperisone + 10 mg/kg dextromethorphan | 79 | 6 | 10 |

Enhancement of efficacy of 40 and 60 mg/kg tolperisone by co-administration of 10 mg/kg dextromethorphan, which is ineffective by itself. Dextromethorphan (10 mg/kg) significantly increased the efficacy of 40 mg/kg and 60 mg/kg tolperisone (p<0.001 and p<0.01).

### Spinal reflex inhibitory action, in vitro

Our experiments were performed according to the methods described by Otsuka and Konishi (Otsuka.and Konishi, Nature 1974. 252, 733.), with slight modifications (Kocsis et al., Brain Res. Bull. 2003. 60, 81.).

Tolperisone or eperisone (chemical name: 2-mehyl-1-(4-ethylphenyl)-3-(1-piperidinyl)-1-propanone), which has a similar structure and pharmacological effect to tolperisone, displayed significant reflex inhibitory activity in the hemisected spinal cord preparation, in vitro. This experiment is suitable for testing central muscle relaxant activity. In the tremor test the efficacy of drugs may be considerably influenced by metabolic and pharmacokinetic factors, therefore we used an in vitro model to investigate the pharmacodynamic interaction between the components of the combination without the interference of the above factors.

Our studies revealed that dextromethorphan alone has a reflex inhibitory activity. We determined the dose-response curve for monosynaptic reflex inhibition of dextromethorphan alone, and in the presence of tolperisone or eperisone. In the control study we measured the effect of dextromethorphan at 0.25; 0.5 and 1 µM. In other experiments we added tolperisone (25 µM) or eperisone (20 µM) to the perfusion solution, and taking the equilibrium responses in these conditions as references, we measured the reflex inhibitory action of dextromethorphan. We found, that dextromethorphan co-administrated with tolperisone is four-fold more effective than alone. The potentiating effect of eperisone has been even more expressed (see Table 4.).

We also demonstrated the synergistic effect by using a converse administration schedule, i.e. we measured tolperisone alone and in the presence of 0.25 µM dextromethorphan, and compared the two dose-response curves (see: Table 5.).

**Table 4.**

| | Dextromethorphan | | | Dextromethorphan with 25 µM tolperisone | | | Dextromethorphan with 20 µM eperisone | | |
|---|---|---|---|---|---|---|---|---|---|
| Concentration of dextromethorphan (µM) | Inhibition (%) | S.E. M. | N | Inhibition (%) | S.E.M. | N | Inhibition (%) | S.E.M. | N |
| 0.25 | 20.73 | 5.27 | 5 | 36.24 | 8.51 | 5 | 56.61 | 8.49 | 4 |
| 0.5 | 36.59 | 8.71 | 5 | 59.41 | 8.06 | 5 | 77.97 | 4.67 | 4 |
| 1 | 40.94 | 20.35 | 3 | 78.22 | 0.86 | 3 | 85.09 | 3.01 | 4 |

Reflex inhibitory activity of dextromethorphan alone and in the presence of tolperisone or eperisone. Co-administration of tolperisone decreased the IC₅₀ value of dextromethorphan by about 75% (IC₅₀ of dextromethorphan alone: 1.56 µM; dextromethorphan in the presence of 25 µM tolperisone: 0.38 µM), i.e. multiplied the efficacy of the drug by a factor of four. The difference between the two dose-response curves is significant (ANOVA, Duncan's multiple range test, p=0.003259). Co-administration of eperisone also decreased the IC₅₀ value of dextromethorphan. (IC₅₀ of dextromethorphan with eperisone: 0.19 µM). The difference compared to the control curve is also significant. (ANOVA, Duncan's multiple range test, p=0.000198).

**Table 5.**

| | Tolperisone | | | with 0.25 µM dextromethorphan | | |
|---|---|---|---|---|---|---|
| Concentration of tolperisone (µM) | Inhibition (%) | S.E.M. | N | Inhibition (%) | S.E.M. | N |
| 25 | 26.97 | 6.35 | 4 | 71.23 | 4.42 | 4 |
| 50 | 48.61 | 8.72 | 4 | 84.71 | 3.47 | 4 |
| 100 | 70.30 | 8.74 | 4 | - | - | - |

Reflex inhibitory activity of tolperisone alone, and in the presence of dextromethorphan . The presence of dextromethorphan decreased the IC₅₀ of tolperisone by nearly 80% (IC₅₀ of tolperisone alone: 52.4 µM; IC₅₀ of tolperisone in the presence of dextromethorphan: 11.4 µM; sigmoid fitting), namely dextromethorphan multiplied the efficacy of tolperisone by a factor of five. The difference between the two curves is significant. (ANOVA, Duncan's multiple range test, p=0.000189).

The above mentioned results unambiguously prove the presence of a potentiating pharmacodynamic interaction between tolperisone and dextromethorphan.

### B./

### Analgesia studies

### Rat allodynia model

In our experiments, we applied the Bennet-Xie model (G.J. Bennett, Y.-K. Xie, Pain 33: 87-107; 1988) to test the effect of the combination against neuropathic pain. We measured the threshold of the pain reaction with von Frey filaments calibrated to 0.9, 1.2, 1.8, 3.6, 5.4, 6.9, 8.9, 12 and 20 g. In those cases when the animal showed no reaction to 20 g, we assigned a threshold value of 21 g.

In this allodynia model, tolperisone had a significant analgesic effect. Dextromethorphan at a dose of 10 mg/kg, i.p. was practically ineffective when applied alone, but in combined treatment it increased the effectiveness of tolperisone considerably (Table 6.).

**Table 6.**

| | Control N=8 | | Dextromethorphan 10mg/kg ; N=7 | | Tolperisone 20mg/kg ; N=7 | | Combination N=7 | |
|---|---|---|---|---|---|---|---|---|
| | Threshold value (g) | | | | | | | |
| Time (min) | mean | S.E.M. | mean | S.E.M. | mean | S.E.M. | mean | S.E.M. |
| 0 | 2.92 | 0.23 | 3.26 | 0.52 | 3.6 | 0.56 | 3.86 | 0.47 |
| 10 | 5.21 | 0.40 | 7.57 | 0.75 | 14.89 | 2.57 | 19.71 | 1.29 |
| 20 | 3.94 | 0.27 | 6.03 | 0.83 | 10.31 | 2.60 | 18.2 | 2.14 |

Anti-allodynic (analgesic) effect of intraperitoneally administered tolperisone alone, and co-administered with dextromethorphan. There is a significant difference between the groups at 10 and 20 minutes (Kruskal-Wallis ANOVA, 0 min: p=0.9135; 10 min: p=0.0004; 20 min: p=0.0024). At its peak effect (10 min) dextromethorphan did not cause a significant effect (p=0.07; Mann-Whitney U test), while tolperisone (p=0.0059; Mann-Whitney U test) and its combination with dextromethorphan (p=0.00031; Mann-Whitney U test) did. The effect of the combination was significantly stronger than that of tolperisone administered alone (p=0.026; Mann-Whitney U test).

### C./

### Discussion of pharmacological results

Results of the tremor test prove that ineffective doses of dextromethorphan can increase the tremor inhibitory (i.e. muscle relaxant, antispastic) activity of tolperisone. Dextromethorphan when co-administered with tolperisone at ratios from 1:19 to 1:4 significantly increased the effect of tolperisone. We obtained similar results in our in vitro reflex test; dextromethorphan in the presence of tolperisone had higher reflex inhibitory activity, than alone, and the same potentiating effect was also demonstrated when a converse administration schedule was applied.

Investigating the analgesic effect of the combination in an allodynia test, an ineffective dose of dextromethorphan significantly increased the analgesic effect of tolperisone. In this case the ratio of dextromethorphan and tolperisone was 1:2.

Our studies have proved, that a potentiating interaction exists between the components of the pharmaceutical combination of dextromethorphan with tolperisone (and with eperisone, which has similar structure and pharmacological effects), in a wide dose range (ratios from 1:2 to 1:20). This potentiating effect is bigger than an additive effect, and it may have a big advantage in human therapy.

### D./

### Clinical experiences

Clinical experiences with only a small number of patients indicate a potential therapeutical benefit of using the tolperisone:dextromethorphan 5:1 fixed-dose combination for different neurological indications.

Our clinical studies were carried out with 14 patients, presenting chronic pain syndrome for at least three months. Patients received 150 mg tolperisone and 30 mg dextromethorphan three times per day. The duration of the treatment varied between 1 and 3 weeks. The combination therapy resulted in a reduction in average and maximum pain intensity in 12 patients, while changes to the opposite direction never occurred. There were no major side effects necessitating discontinuation of the treatment.

Based on patients' self-reports, pain relief started several days after the beginning of the treatment. The etiological backgrounds included radiculopathy, mitochondrial myopathy, diabetes and paraneoplastic polineuropathy, fibromyalgia, stiff-man syndrome.

According to our invention, the combination offers the following advantages:

The treatment for certain diseases accompanied by pain or spasticity is not resolved in medical practice. According to the above mentioned findings, the fix-dose combination of tolperisone and dextromethorphan can lead to a new and more effective therapy in the treatment of diseases accompanied by pain or spasticity.

The pharmaceutical combination of our invention had a significant antispastic and analgesic activity in animal tests, in vivo and in vitro, which is bigger than the sum of the effect of the components measured separately (i.e. the synergism is supra-additive). This indicates that pharmaceutical combinations of tolperisone-dextromethorphan or eperisone-dextromethorphan can be prepared, with the same antispastic efficacy but with lower metabolic load (using lower doses), or is more effective (at the same doses) than tolperisone or eperisone when administered alone.

Animal test results point at a new indication of the combination; analgesia, which until now was not among the indications of either tolperisone or dextromethorphan. Therefore the combination can be used not only for the treatment of spastic states, which can not be treated by monotherapy with any of the two compounds, but also for the treatment of pain syndromes with various etiological backgrounds.

The suitable range of the ratios of the two components depends on the metabolic features of the tested species. The ratios of the combination can be between 20:1 and 2:1, wherein tolperisone and eperisone is present at the higher dose. In humans we obtained encouraging results with a ratio of 5:1 in the tolperisone-dextromethorphan combination.

Dextromethorphan and tolperisone (or eperisone) do not show incompatibility, therefore a pharmaceutical composition - containing two active ingredients in determined doses - can be prepared with pharmaceutically acceptable carriers. According to the invention, the formulation of our pharmaceutical composition is demonstrated with the following examples:

### EXAMPLE 1

We homogenize and granulate the listed ingredients. The quantity of the prepared granulation is sufficient for the preparation of 10 units of 1 g tablets.

| | |
|---|---|
| Dextromethorphan | 0.3 g |
| Tolperisone | 1.5 g |
| Lactose | 1.05 g |
| Microcrystalline cellulose | 0. 55 g |
| PVP (Polyvinylpirrolydone) | 0.16 g |
| Mg stearate | 0.4 g |
| UAP (ultraamylopectin) | 6.4 g |

### EXAMPLE 2

We homogenize and granulate the listed ingredients. The quantity of the prepared granulation is sufficient for the preparation of 10 units of 1 g tablets.

| | |
|---|---|
| Dextromethorphan | 0.15 g |
| Tolperisone | 1.5 g |
| Lactose | 1.05 g |
| Microcrystalline cellulose | 0. 55 g |
| PVP (Polyvinylpirrolydone) | 0.16 g |
| Mg stearate | 0.4 g |
| UAP (ultraamylopectin) | 6.55 g |

### EXAMPLE 3

We homogenize and granulate the listed ingredients. The quantity of the prepared granulation is sufficient for the preparation of 10 units of 1.5 g tablets.

| | |
|---|---|
| Dextromethorphan | 0.3 g |
| Tolperisonee | 3.0 g |
| Lactose | 1.05 g |
| Microcrystalline cellulose | 0. 55 g |
| PVP (Polyvinylpirrolydone) | 0.16 g |
| Mg stearate | 0.4 g |
| UAP (ultraamylopectin) | 9.9 g |

### EXAMPLE 4

We homogenize and granulate the listed ingredients. The quantity of the prepared granulation is sufficient for the preparation of 10 units of 1 g tablets.

| | |
|---|---|
| Dextromethorphan | 0.6 g |
| Tolperisone | 1.5 g |
| Lactose | 1.05 g |
| Microcrystalline cellulose | 0. 55 g |
| PVP (Polyvinylpirrolydone) | 0.16 g |
| Mg stearate | 0.4 g |
| UAP (ultraamylopectin) | 6.1 g |

## Claims

1. Pharmaceutical combination for the treatment of spasticity and/or pain **characterized by** that the combination contains as active ingredient 70-95% w/w compound of formula I, wherein R represents a methyl or ethyl group, and 5-30 % w/w. dextromethorphan (chemical name: (+/-)-3-methoxi-17-methylmorphinan).

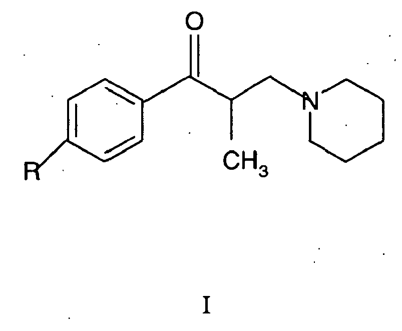

2. A pharmaceutical combination according to claims 1, wherein the compound of formula I, wherein R represents a methyl group is present in an amount of 80 to 90% w/w and dextromethorphan is present in an amount of 10 to 20% w/w.

3. A pharmaceutical composition as claimed in any one of the preceding claims wherein a unit of the composition contains 150 mg tolperisone and 30 mg dextromethorphan in admixture with pharmaceutically acceptable carriers.

4. A pharmaceutical composition comprising a therapeutically effective amount of a combination as claimed in any one of claims 1-3 wherein the composition is for oral administration.

5. Use of a combination as claimed in claim 1, for the manufacture of a medicament for the prevention and/or treatment of spasticity and/or pain.

## Patentansprüche

1. Pharmazeutische Kombination für die Behandlung von Spastik und/oder von Schmerzen, **gekennzeichnet** darin, daß die Kombination als Wirkstoff 70 bis 95 % G/G der Verbindung der Formel (I) enthält, worin R eine Methyl- oder Ethylgruppe bedeutet, und 5 bis 30 % G/G Dextromethorphan (chemischer Name: (+/-)-3-Methoxy-17-methylmorphinan)

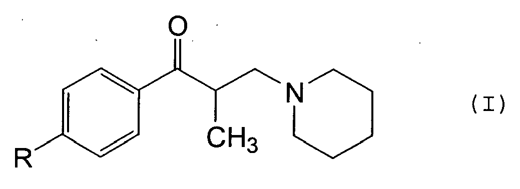

2. Pharmazeutische Kombination gemäß Anspruch 1, wobei die Verbindung der Formel (I), worin R eine Methylgruppe bedeutet, in einer Menge von 80 bis 90 % G/G und Dextromethorphan in einer Menge von 10 bis 20 % G/G vorliegt.

3. Pharmazeutische Zusammensetzung gemäß einem der vorstehenden Ansprüche, wobei eine Einheit der Zusammensetzung 150 mg Tolperison und 30 mg Dextromethorphan in Vermischung mit pharmazeutisch annehmbaren Trägern enthält.

4. Pharmazeutische Zusammensetzung, umfassend eine therapeutisch effektive Menge einer Kombination gemäß einem der Ansprüche 1 bis 3, wobei die Zusammensetzung für die orale Verabreichung gedacht ist.

5. Verwendung einer Kombination gemäß Anspruch 1 für die Herstellung eines Medikaments für die Prävention und/oder Behandlung von Spastik und/oder Schmerz.

## Revendications

1. Combinaison pharmaceutique pour le traitement de l'hypertonie spastique et/ou de la douleur **caractérisée en ce que** la combinaison contient comme ingrédient actif 70-95 % p/p de composé de formule I, dans laquelle R représente un groupe méthyle ou éthyle, et 5-30 % p/p de dextrométhorphane (nom chimique (+/-)-3-méthoxy-17-méthylmorphinane).

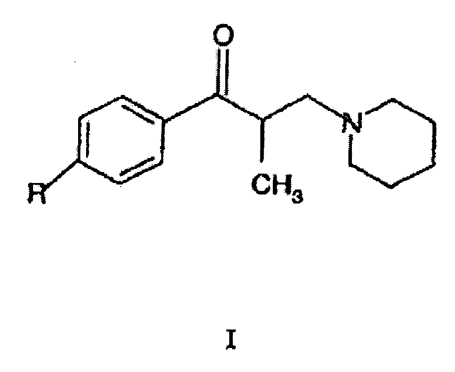

2. Combinaison pharmaceutique selon la revendication 1, dans laquelle le composé de formule I, dans lequel R représente un groupe méthyle, est présent en une quantité de 80 à 90 % p/p et le dextrométhorphane est présent en une quantité de 10 à 20 % p/p.

3. Composition pharmaceutique selon l'une quelconque des revendications précédentes dans laquelle une unité de la composition contient 150 mg de tolpérisone et 30 mg de dextrométhorphane en mélange avec des porteurs pharmaceutiquement acceptables.

4. Composition pharmaceutique comprenant une quantité thérapeutiquement efficace d'une combinaison selon l'une quelconque des revendications 1-3, dans laquelle la composition est destinée à l'administration orale.

5. Utilisation d'une combinaison selon la revendication 1, pour la fabrication d'un médicament pour la prévention et/ou le traitement de l'hypertonie spastique et/ou de la douleur.
